# EUROPEAN PATENT APPLICATION

(11) **EP 2 574 316 A2**
(43) Date of publication of application: **03.04.2013**
(21) Application number: 12006830.9
(22) Date of filing: 01.10.2012
(51) Int. Cl.: A61F 2/95

(54) **Edgeless unions of concentric members**

(30) Priority: 30.09.2011 US 201161542151 P
(71) Applicant: Cordis Corporation, Bridgewater, NJ 08807 (US)
(72) Inventor: Young, Joshua, Montclair, NJ (US)
(74) Representative: Diehl & Partner GbR

(57) **Abstract**

Leading edges (204) of concentric members (202) are desirably hidden in unions of two concentric members (102, 202) intended for advancement through the vasculature for vasculature implant delivery. Grooves (104) or impressions on the inner concentric member (102) provide spaces for the leading edge (204) of the distal tip of the outer concentric member (202) to create edgeless unions of two concentric members (102, 202) for safe and unimpeded advancement and operation of vascular implant delivery devices which include the two concentric members (102, 202).

## Description

### BACKGROUND

. 1. Technical Field

. The invention relates to the field of medical devices, and more particularly a vascular implant delivery system.

. 2. Related Devices and Methods

. Vascular disease is a leading cause of premature mortality in developed nations. Treatment of vascular disease may include implantation of tissue supporting stents or prosthetic vasculature, e.g., grafts, stent-grafts, etc., which are delivered through the vasculature at a reduced dimension for ease of navigation in, and reduced chance of injury to, the tortuous vasculature from entry point to the diseased location. These vascular implant delivery devices typically include an elongated shaft around which the vascular implant is disposed at a distal end, that is the end furthest from the medical professional implanting the vascular implant. Such shafts may have variable designs as best suited to deliver the vascular implant from the point of entry to the vasculature to the intended implantation site. Some delivery devices further include additional features such as soft tips on the distal ends of the elongated shafts, sheaths or outer members disposed about much of the length of the elongated shaft and about the vascular implant, and various features on the proximal end, that is, the end closest to the medical professional to perform varied functions, e.g., release of dye or other visualization agent, valved access to a lumen running through the elongated shaft for inserting a guide wire, sealed attachment of a pressurized fluid to inflate balloons at the distal end, or other mechanisms involved in the controlled delivery of the vasculature to its intended site.

. It is desirable to have a smooth transition at any union of two concentric members, such as at the tip of inner and outer members of vascular implant delivery systems, that is expected to interact with the patient's vasculature to prevent inadvertent injury to the vessel from insertion and tracking of the device to the target location. Such interactions with the vessel wall can cause vessel spasm, damage to health intima or even vessel perforation or dissection.

. Secondly any edge created by such a union might be a liability for any procedure that introduces more than one stent or stent-graft. After delivery of the first implant, the delivery system of the second implant is required to be inserted in to the lumen of the deployed implant to create an overlap. Upon introduction of the second delivery system any edge on the delivery system that is exposed can potentially snag on the deployed first implant. Unintended interactions, such as snagging, may cause problems with the placement of the first implant, interaction of the first implant and the vessel wall or fatigue of the first implant itself.

. SUMMARY

. In some embodiments according to the invention, grooves or impressions made in the outer surface of an inner member of two concentric member hides the leading edge of the tip of the outer concentric member allowing for a more continuous unified transition. These grooves or impression may be angled to match the outer tip design or may be generic grooves that are intended just to hide the edge of the outer tip. There can be a single groove or there could also be more than one groove.

. These and other features, benefits, and advantages of the present invention will be made apparent with reference to the following detailed description, appended claims, and accompanying figures, wherein like reference numerals refer to structures that are either the same structures, or perform the same functions as other structures, across the several views.

. BRIEF DESCRIPTION OF THE FIGURES:

. The figures are merely exemplary and are not meant to limit the present invention.

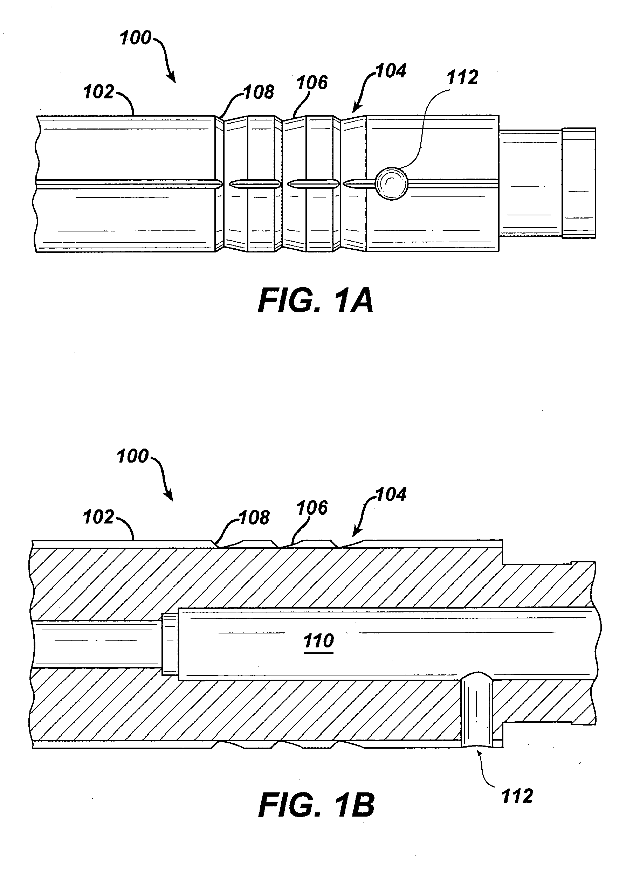

. FIG. 1A is a top view of a three dimension rendering of an inner member, near the distal tip.

. FIG. 1B is a top view of the inner member of FIG. 1A.

. FIG. 1C is a cross-sectional view of the inner member of FIGS. 1A and 1B.

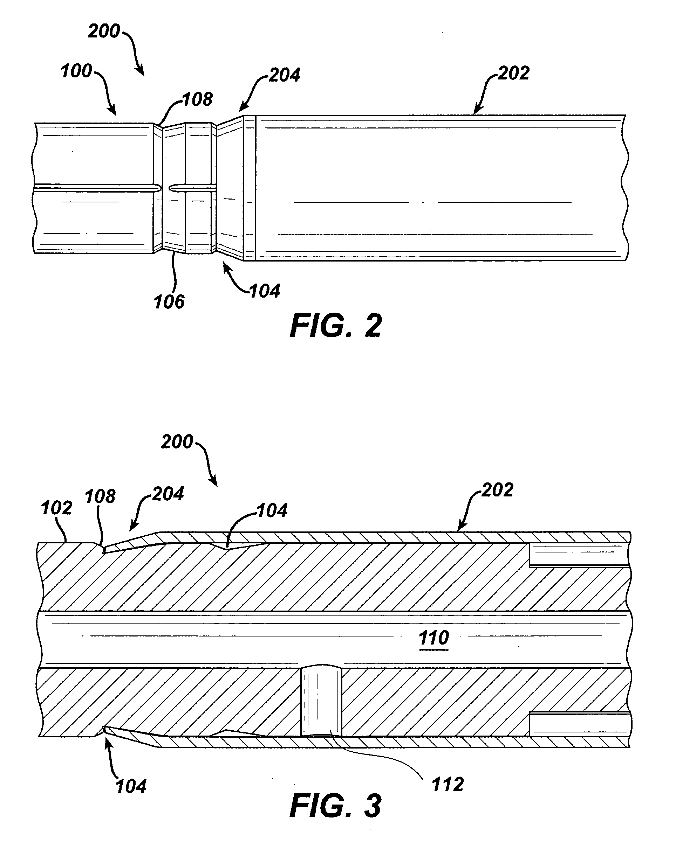

. FIG. 2 is a top view of the inner member of FIG. 1B assembled with a concentric outer member, showing the union of the distal tip of the concentric outer member with the inner member.

. FIG. 3 is a cross sectional view of an enlarged portion of the assembly of FIG. 2.

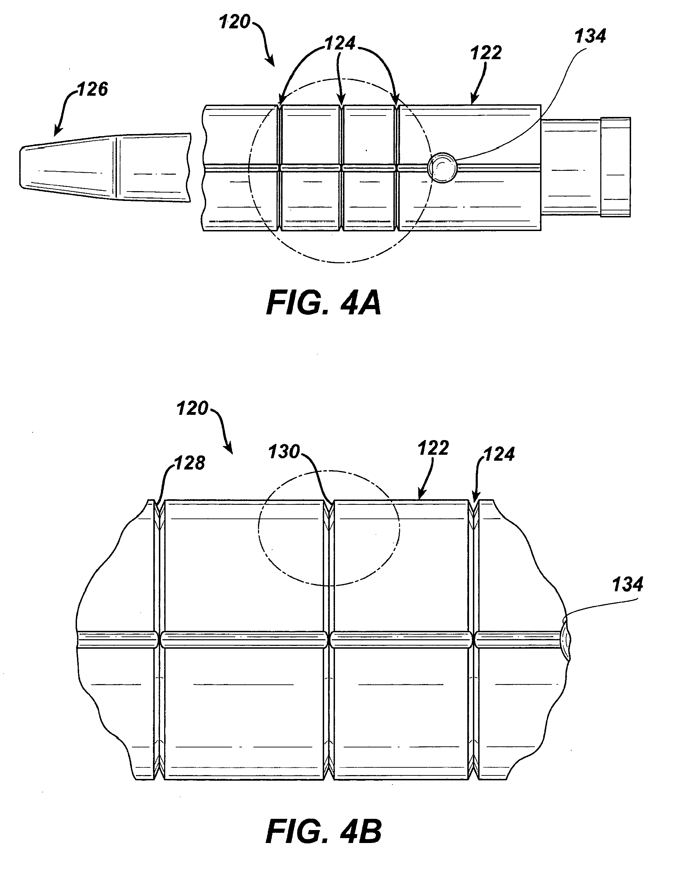

. FIG. 4A is a top view of a three dimensional rendering of a distal portion and tip of an inner member with a second embodiment of grooves.

. FIG. 4B is a top view of an enlarge portion of FIG. 4A.

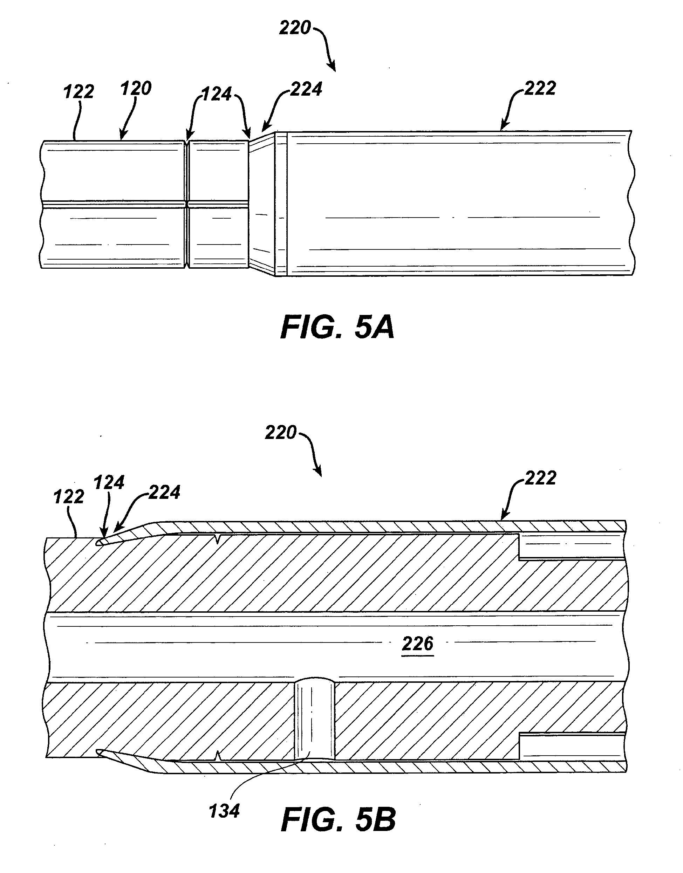

. FIG. 5A is a top view of the inner member of FIG. 3 assembled with a concentric outer member, showing the union of the distal tip of the concentric outer member with the inner member.

. FIG. 5B is a cross-sectional view of an enlarged portion of the assembly of FIG. 5A.

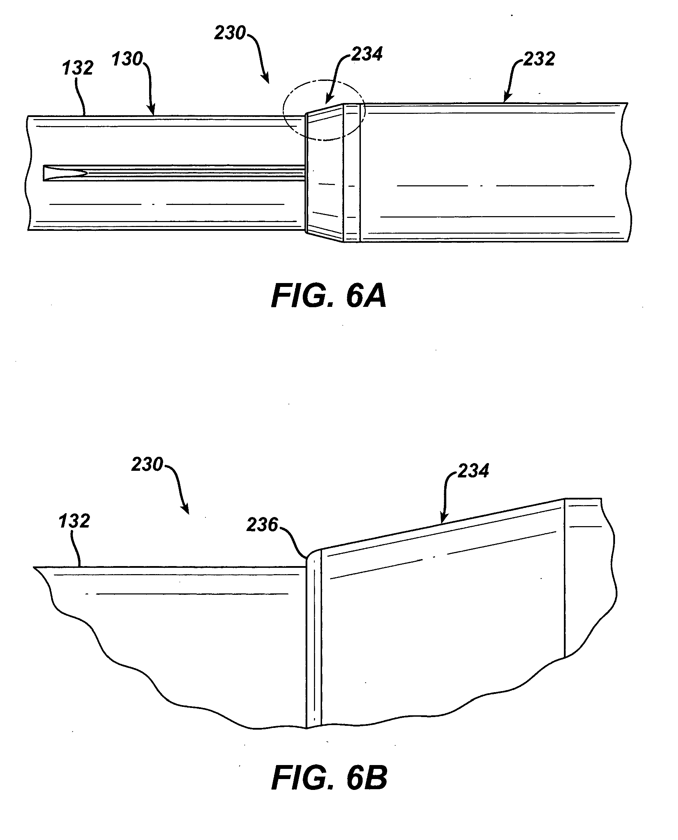

. FIG. 6A is a top view of an assembly of yet a third inner member, without circumferential grooves, assembled with another embodiment of an outer concentric member, showing the union of the distal tip of the concentric outer member with the inner member.

. FIG. 6B is an enlargement of a portion of FIG. 6A.

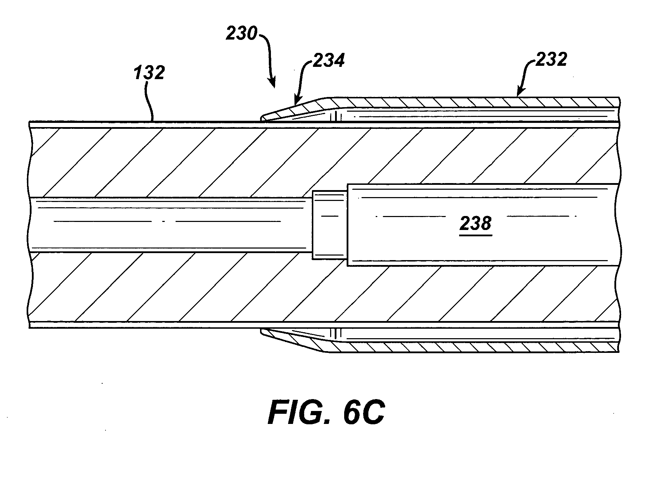

. FIG. 6C is a cross section of an enlarged portion of FIG. 6A.

. DETAILED DESCRIPTION

. An edgeless union of two concentric members is a feature that is designed to be used where two tips (an inner and outer member) come together and form a union. Without the benefit of embodiments of this invention, a solid tubular inner tip would have a tip of a concentric outer member resting on its outer groove-less surface creating a bump/step transition ,such as depicted in FIGS. 6A, 6B, and 6C, that can lead to tissue damage and/or procedural issues due to the tip of the concentric outer member snagging.

. Edgeless unions of concentric members may include grooves, such as those depicted on inner member in FIGS. 1A-1C and 4A -4B to hide the edge of the tip of the concentric outer member allowing for a more continuous, unified transition, as depicted in FIGS. 2, 3, and 5A-5B instead of the bump or step transition. In some embodiments, grooves may be angled to match the outer tip design, such as depicted in FIGS. 1A-3, or may be generic grooves, such as those depicted in FIGS. 4A-5B, that are intended just to hide the edge of the tip of the outer concentric member. In some embodiments there is a single groove. In some embodiments, more than one groove can be included to provide a length of the inner member along which the edge of the concentric outer member will "land" upon final assembly, taking into account length tolerances of the inner and outer concentric members.

. In some embodiments, the depth, width (longitudinal length) and angle of these grooves are enough to allow hiding of the edge but still allow for linear retraction of the inner member back through the concentric outer member.

. One example is illustrated in FIGS. 6A through 6C where the leading edge of the outer member is given a radius. However, this example fails to hide the edge and just simply attempts to make the edge less sharp.

. A second example as illustrated in FIGS. 6A-6C includes drawing down the diameter of the tip of the concentric outer member. This example puts a taper on some distal portion of the outer member in an attempt to have an angle of transition to the outer member body. This example works well for preventing of "fish mouthing" (a condition of the outer member becoming oval and gapping on opposite sides, resembling an open fish mouth) because of tight conformance of the outer member to the inner member and does provide an angle of transition instead of a true step transition. However this taper does not hide the edge that is created going from a smaller diameter of the outer diameter of the inner member to the larger diameter of the inner diameter of the concentric outer member on it.

. Yet another example of an edgeless union is an inner member with a smaller outer diameter on the proximal end and a larger outer diameter on the distal end (much like an arrow) to essentially create a plug when inserted into the outer member. However, in these examples, the tip of the concentric inner member is not made to be withdrawn back through the concentric outer member; it would not be able to be retracted back due to large sharp change in diameter.

. Other embodiments of edgeless unions of concentric members have impressions (circumferential angulated or non angulated impressions) in the outer surface of the inner member that will allow the concentric outer member to conform to it, thereby hiding the edge while still achieving a tight conformance to the inner member and allowing the retraction of the inner member back through the outer. It is counterintuitive that impressions are put onto the inner member tip making it less smooth in order to achieve a smoother transition as an assembly when mated with the outer member. In some embodiments, one circumferential angulated or non angulated impression will be on the inner concentric member. In some embodiments, any number of circumferential angulated or non angulated impressions may be made in the inner concentric member to allow easier assembly in manufacturing.

. Embodiments including the impressions on the inner member could also allow physicians the ability to initiate deployment, decide to abort deployment to reposition or retract completely. In doing so the physician would just retract to the next impression which would ensure the edge is hidden and the physician may maneuver the delivery system with the same performance as he or she did during initial insertion.

. FIG. 1A is a top view of a three dimension rendering of an inner member 100, near the distal tip (not shown). Inner member 100 has an outer diameter with a cylindrical outer surface 102 in which three circumferential grooves 104 are formed. Each groove 104 has two opposing tapered faces, tapered face 106 with a decreasing diameter along the proximal to distal length, and tapered face 108 with an increasing diameter along the proximal to distal length. The tapered face 108 has a much steeper taper, such that the groove is not symmetric.

. FIG. 1B is a top view of the inner member of FIG. 1A.

. FIG. 1C is a cross-sectional view of the inner member of FIGS. 1A and 1B. It may be seen that in this embodiment, inner member 100 has a lumen 110 therethrough.

. FIG. 2 is a top view of inner member 100 of FIG. 1B assembled (assembly 200) with a concentric outer member 202, showing the union of distal tip 204 of concentric outer member 202 with inner member 100. It may be seen that distal tip 204 is hidden within the second of the three grooves 104.

. FIG. 3 is a cross sectional view of an enlarged portion of the assembly of FIG. 2, and illustrates the leading edge of distal tip 204 within groove 104, resulting in an edgeless union of two concentric members.

. FIG. 4A is a top view of a three dimensional rendering of a distal portion and tip 126 of an inner member 120 with a second embodiment of grooves 124 in outer cylindrical surface 122 of inner member 120. Inner member 120 has three circumferential grooves in this embodiment, spaced unevenly along the longitudinal length.

. FIG. 4B is a top view of an enlarge portion of FIG. 4A illustrating that grooves 124 have two opposing tapered faces 128 and 130. The taper of tapered face 128 is an increasing diameter in the distal direction and the taper of tapered face 130 is a decreasing diameter in the distal direction, and the tapers are the same, such that the groove 124 is symmetric.

. FIG. 5A is a top view of the inner member 120 of FIG. 3 assembled (assembly 220) with a concentric outer member 222, showing the union of the distal tip 224 of the concentric outer member 222 with the second groove 124 of inner member 120.

. FIG. 5B is a cross-sectional view of an enlarged portion of the assembly of FIG. 5A, which illustrates that inner member 120 has a lumen 226 therethrough.

. FIG. 6A is a top view of an assembly 230 of yet a third inner member 130, without circumferential grooves, assembled with another example of an outer concentric member 232, showing the union of the distal tip 234 of the concentric outer member 232 with outer cylindrical surface 132 of the inner member 130.

. FIG. 6B is an enlargement of a portion of FIG. 6A illustrating the tapered distal tip 234 ending in an exposed leading edge 236.

. FIG. 6C is a cross section of an enlarged portion of FIG. 6A illustrating that in this example, inner member 130 has a lumen 238 therethrough and the step or bump transition presented by the leading edge of the tapered distal tip 234 of outer concentric member 232.

. Aspects of the present invention have been described herein with reference to certain exemplary or preferred embodiments. These embodiments are offered as merely illustrative, not limiting, of the scope of the present invention. Certain alterations or modifications possible include the substitution of selected features from one embodiment to another, the combination of selected features from more than one embodiment, and the elimination of certain features of described embodiments. Other alterations or modifications may be apparent to those skilled in the art in light of instant disclosure without departing from the scope of the present invention, which is defined solely with reference to the following appended claims.

. To summarize, the present application discloses a system of concentric inner and outer members, the system comprising an inner tubular member having a distal end and a proximal end and one or more circumferential grooves in its outer surface; and an outer tubular member, concentric with the inner tubular member and having a distal end and a proximal end, the distal end having a leading distal edge within one of the one or more circumferential grooves of the inner tubular member, thereby forming an edgeless union of the distal end of the outer tubular member with the inner tubular member when assembled. The system may be a vascular implant delivery system. The inner member may have only one, or two or three or more circumferential grooves. When there are at least three grooves, the spacings between the at least three grooves may be uneven, e.g. differ by 5-100% of the smallest of the spacings, or by 10-50%. The circumferential grooves may be non-angulated or angulated, in which case the grooves may have different distal and proximal taper angles ("distal" here meaning "located nearer to the system's distal tip" 126 in Fig. 4A). E.g., the distal taper angle is at least 20°, or at least 30°, or at least 45° with respect to the longitudinal direction. In this case, the proximal taper angle may be at most 20°, or at most 30°, or at most 45° with respect to the longitudinal direction, and independently is at least 10°, or at least 20°, or at least 30° less than the distal taper angle. A total depth of the one or more grooves may be at least as large or larger than a thickness of the wall of the outer tubular member at its distal end. The inner tubular member may have a hole communicating the inner lumen with the periphery, wherein the hole is located distally of the one or more grooves, i.e. between the connected end and the last groove.

. The present application also discloses the use of a tubular member having one or more grooves in its outer surface, for forming a joint vascular implant assembly, wherein the tubular member may be the inner tubular member as defined above. The joint may be formed by coaxially connecting the tubular member with an outer tubular member having a drawn-in distal end.

## Claims

1. A system of concentric inner and outer members, the system comprising:
an inner tubular member (100; 120) having a distal end and a proximal end and one or more circumferential grooves (104, 106, 108; 124, 128, 130) in its outer surface (102; 122); and
an outer tubular member (202; 222), concentric with the inner tubular member (100; 120) and having a distal end and a proximal end, the distal end having a leading distal edge (204; 224) within one of the one or more circumferential grooves (104, 106, 108; 124, 128, 130) of the inner tubular member (100; 120),
thereby forming an edgeless union of the distal end of the outer tubular member (202; 222) with the inner tubular member (100; 120) when assembled.

2. The system of claim 1, wherein the system is a vascular implant delivery system (200).

3. The system of claim 1 or 2, the inner member having two or three or more circumferential grooves (104, 106, 108; 124, 128, 130).

4. The system of claim 3, wherein there are at least three grooves (104, 106, 108; 124, 128, 130), and the spacings between the at least three grooves are uneven, e.g. differ by 5-100% of the smallest of the spacings.

5. The system of claim 1 or 2, the inner member having only one circumferential groove.

6. The system of one of claims 1 to 5, wherein the circumferential grooves are non-angulated.

7. The system of one of claims 1 to 5, wherein the circumferential grooves (104, 106, 108; 124, 128, 130) are angulated.

8. The system of claim 7, wherein the grooves (104, 106, 108) have different distal (108) and proximal (106) taper angles.

9. The system of claim 8, wherein the distal taper (108) angle is at least 20°, or at least 30°, or at least 45° with respect to the longitudinal direction.

10. The system of claim 9, wherein the proximal taper (106) angle is at most 20°, or at most 30°, or at most 45° with respect to the longitudinal direction, and independently is at least 10°, or at least 20°, or at least 30° less than the distal taper (108) angle.

11. The system of one of claims 1 to 10, wherein a total depth of the one or more grooves (104, 106, 108; 124, 128, 130) is at least as large or larger than a thickness of the wall of the outer tubular member (202; 222) at its distal end.

12. The system of one of claims 1 to 11, the inner tubular member (100; 120) having a hole communicating the inner lumen (110; 226) with the periphery, wherein the hole is located distally of the one or more grooves (104, 106, 108; 124, 128, 130).

13. Use of a tubular member (100; 120) having one or more grooves (104, 106, 108; 124, 128, 130) in its outer surface (102; 122), for forming a joint vascular implant assembly (200; 220).

14. The use of claim 13, wherein the tubular member is the inner tubular member (100; 120) as defined in one of claims 1 to 12.

15. The use of claim 13 or 14, wherein the joint is formed by coaxially connecting the tubular member (100; 120) with an outer tubular member (202; 222) having a drawn-in distal end (224).
